# EUROPEAN PATENT APPLICATION

(11) **EP 3 564 959 A1**
(43) Date of publication of application: **06.11.2019**
(21) Application number: 19172215.6
(22) Date of filing: 02.05.2019
(51) Int. Cl.: G16H 20/10, G16H 40/60

(54) **CODE SCANNING FOR DRUG DELIVERY**

(30) Priority: 02.05.2018 US 201815969295
(71) Applicant: Insulet Corporation, Acton MA 01720 (US)
(72) Inventor: COLLERAN, David, Medfield, MA Massachusetts 02052 (US); NAZZARO, David, Groveland, MA Massachusetts 01834 (US); O'CONNOR, Jason, Acton, MA Massachusetts 01720 (US); MALEK, Aiman, La Jolla, CA California 92037 (US)
(74) Representative: Vossius & Partner Patentanwälte Rechtsanwälte mbB

(57) **Abstract**

A drug delivery system is provided. The drug delivery system can include a drug delivery device configured to store and deliver a drug to a user and a drug delivery management device for managing operation of the drug delivery device. The drug delivery management device can determine information relating to the drug that is to be provided to the user. The information relating to the drug can be provided by an indicator coupled to a container configured to store the drug. The drug delivery management device can determine a software program for controlling operation of the drug delivery management device based on the determined information relating to the drug. Other embodiments are disclosed and described.

## Description

### TECHNICAL FIELD

Embodiments generally relate to medication delivery. More particularly, embodiments relate to configuration of drug delivery devices and/or drug delivery management devices.

### BACKGROUND

Many conventional drug delivery systems include a drug delivery device paired with a remote management device. Typically, the drug delivery device is worn by a user and includes a drug to be delivered to the user. The remote management device is used to control operation of the wearable drug delivery device to provide automated delivery of the stored drug to the user.

For many conventional drug delivery systems, the remote management device is configured to operate with only a specific type of drug delivery device and/or only a specific type of drug. As a result, a new or different management device is required when a different drug type or drug concentration is used with the drug delivery device. This requires providers of the drug delivery system to maintain many stock keeping units (SKUs) for at least the management devices, due to the restrictive pairing of the management devices to the drug delivery devices. The costs associated with managing the various SKUs for all drug delivery systems and components thereof can be substantial. A need therefore exists for a drug delivery system and/or a management device for a drug delivery device that can be used across various drug delivery devices, drug types, and/or drug concentrations.

### BRIEF DESCRIPTION OF THE DRAWINGS

FIG. 1 illustrates an exemplary drug delivery system.
FIG. 2 illustrates an exemplary drug container.
FIG. 3 illustrates an exemplary method of operation for a drug delivery system.

### DETAILED DESCRIPTION

This disclosure presents various systems, components, and methods for configuring a drug delivery system and/or a component thereof. Each of the systems, components, and methods disclosed herein provides one or more advantages over conventional systems, components, and methods.

Various embodiments include a drug delivery system. The drug delivery system can include a drug delivery device configured to store and deliver a drug to a user and a drug delivery management device for managing operation of the drug delivery device. The drug delivery management device can determine information relating to the drug that is to be provided to the user. The information relating to the drug can be provided by an indicator coupled to a container configured to store the drug. The drug delivery management device can determine a software program for controlling operation of the drug delivery management device based on the determined information relating to the drug. Other embodiments are disclosed and described.

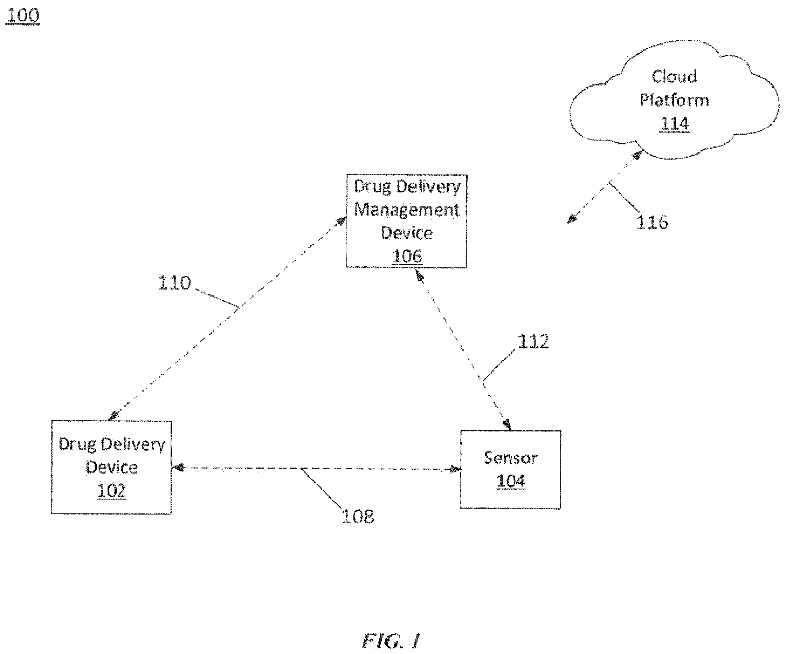

FIG. 1 illustrates an exemplary drug delivery system 100. The drug delivery system 100 can include a drug delivery device 102, a sensor 104, and a drug delivery management device 106. In various embodiments, the drug delivery device 102 can be a wearable device and/or can be attached to the body of a user or patient. For example, the drug delivery device 102 can be directly coupled to the user (e.g., directly attached to a body part and/or skin of the user). The drug delivery device 102 can deliver any therapeutic agent, including any drug or medicine, to the user.

The drug delivery device 102 can include a number of components to facilitate delivery of a drug to the user. In various embodiments, the drug delivery device 102 can include a reservoir for storing the drug, a needle or cannula for delivering the drug into the body of the user, and a pump or other drive mechanism for transferring the drug from the reservoir, through the needle or cannula, into the body of the user. The drug delivery device 102 can also include a power source such as a battery for supplying power to the pump and/or other components of the drug delivery device 102.

The drug delivery device 102 can provide the stored therapeutic agent to the user based on information provided by the sensor 104 and/or information and/or commands from the drug delivery management device 106. In various embodiments, the drug delivery device 102 can be programmed or controlled to automate delivery of the stored drug to the user. In various embodiments, the user can adjust any programmed drug delivery using the drug delivery management device 106 based on, for example, information from the sensor 104. In general, the drug delivery management device 106 can be used to control operation of the drug delivery device 102 to manage delivery of the drug to the user.

In various embodiments, the sensor 104 can be coupled to the user and can provide data on one or more medical conditions and/or physical attributes of the user. The information provided by the sensor 104 can be used to adjust drug delivery operations of the drug delivery device 102 either automatically or based on user manipulation. The drug delivery management device 106 can be used to program or adjust operation of the medical device 102 and/or the sensor 104. The drug delivery management device 106 can be any portable electronic device including, for example, a dedicated controller or a personal electronics device such as a smartphone or tablet.

The drug delivery device 102 and the sensor 104 can communicate over a wireless link 108. The drug delivery device 102 and the drug delivery management device 106 can communicate over a wireless link 110. The sensor 104 and the drug delivery management device 106 can communicate over a wireless link 112. The wireless links 108, 110, and 112 can be any type of wireless link provided by any known wireless standard. As an example, the wireless links 108, 110, and 112 can provide communication links based on Bluetooth, Wi-Fi, a near-field communication (NFC) standard, a cellular standard, or any other wireless protocol.

In various embodiments, the drug delivery system 100 can include a cloud platform 114. The cloud platform 114 can include one or more remote computing platforms (e.g., one or more computer networks, servers, databases, and/or computing devices) that can be used, for example, to store operational data for the drug delivery system 100 and/or direct operation of the drug delivery system 100. For example, data related to the amount of drug delivered to the user over a period of time can be uploaded to the cloud platform 114 for storage or analysis. As another example, a remote service associated with the cloud platform 114 can monitor operation of the drug delivery system 100 and can provide alerts to the user or adjust operation of the drug delivery device 102 in response to detected risks. The cloud platform 114 can communicate with the drug delivery device 102, the sensor 104, and/or the drug delivery management device 106 over a wireless link 116. The wireless link 116 can be any type of wireless communication link including those described above in relation to wireless links 108, 110, and 112.

In various embodiments, the drug delivery device 102 can be a reusable device or a disposable device. In various embodiments, the drug delivery device 102 can be intended for a single use and then discarded. In various embodiments, the drug delivery device 102 can be intended for repeated or multiple use. In various embodiments, the drug delivery device 102 can be filled and/or refilled with a drug by a user. For example, the user can transfer a drug from a cartridge, vial, or other container to the drug delivery device 102 (e.g., using a syringe). As another example, the drug delivery device 102 can be designed to accept a cartridge, vial, or other container containing a drug that the drug delivery device 102 can interact with for expelling the drug for delivery to the user. Accordingly, the drug delivered by the drug delivery device 102 can be provided to the patient preinstalled in the drug delivery device 102 and/or can be provided in a separate container to be used with the drug delivery device 102.

In various embodiments, the drug delivery system 100 can be an insulin drug delivery system. For example, the drug delivery device 102 can be an automated wearable insulin delivery device. In various embodiments, the drug delivery device 102 can be the OmniPod® (Insulet Corporation, Billerica, MA) insulin delivery device as described in U.S. Pat. No. 7,303,549, U.S. Pat. No. 7,137,964, or U.S. Pat. No. 6,740,059, each of which is incorporated herein by reference in its entirety. In various embodiments, the sensor 104 can be a glucose monitor or a continuous glucose monitor (CGM). Glucose monitoring data determined by the sensor 104 can be provided to the drug delivery device 102, the user, and/or the drug delivery management device 106 and can be used to adjust delivery of insulin by the drug delivery device 102. In various embodiments, the management device 106 can be a personal diabetes manager (PDM) and/or a drug delivery manager (DDM). In various embodiments, the drug delivery system 100 can operate according to or can include any of the features or functionalities of the drug delivery systems described in U.S. Patent Application No. 15/359,187, filed November 22, 2016, which is herein incorporated by reference in its entirety.

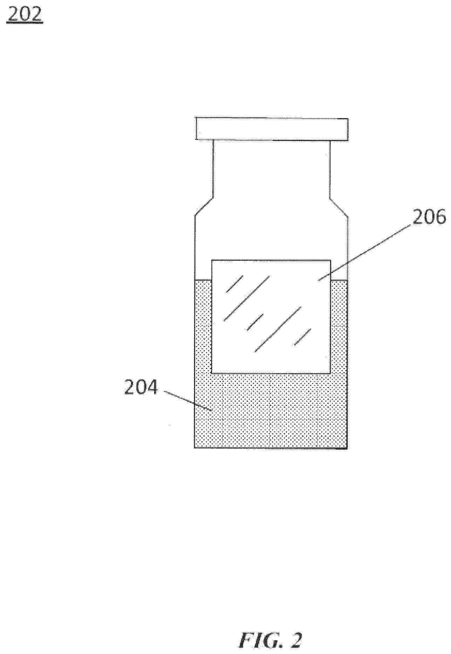

FIG. 2 illustrates an exemplary drug container 202. The drug container 202 can be a drug vial. For example, the drug container 202 can store a liquid drug or other therapeutic agent 204 that can be transferred (e.g., by a syringe) to the drug delivery device 102 for storage and subsequent delivery to the user. The drug container 202 is not limited to being implemented as a drug vial. In various embodiments, the drug container 202 can be an International Organization for Standardization (ISO) drug cartridge (e.g., a 3 mL ISO drug cartridge). Under such a scenario, the drug container 202 as a drug cartridge may be inserted into the drug delivery device 102 to enable the drug delivery device 102 to access and deliver the liquid drug 204.

In various embodiments, the drug container 202 can include an indicator 206. The indicator 206 can include identification information. The identification information can be provided as textual, graphical, or any other visual information. In various embodiments, the indicator 206 can be a drug label. In various embodiments, the indicator can be a bar code or a quick response (QR) code. The indicator 206 can contain or provide an indication of various information relating the drug 204 including, but not limited to, the type of the drug 204, dosage information, expiration information, and patient information. The indicator 206 can be positioned on any portion of the drug container 202.

In various embodiments, the drug delivery device 102 can include a bar code scanner and/or a camera. The bar code scanner can allow the user of the drug delivery management device 106 to scan the indicator 206 (e.g., as a bar code) to capture and interpret information contained in the indicator 206. The camera can allow the user of the drug delivery management device 106 to capture an image of the drug container 202 and/or the indicator 206 for determination of the information contained in the indicator 206. Under either scenario, the drug delivery management device 106 can include one or more devices or mechanisms for receiving the information provided by the indicator 206.

In various embodiments, the drug delivery management device 106 (and/or the drug delivery device 102) can be configured based on information determined from the indicator 206. Configuration of the drug delivery management device 106 can include loading, storing, executing and/or configuring software or other computer-implemented code for directing operation of the drug delivery device 102. The configuration of the drug delivery management device 106 can be based on the type of the drug 204 contained in the drug container 202 as well as other information such as the manufacturer, date of expiration, patient, etc.

In various embodiments, the drug delivery management device 106 can include various versions of software for controlling operation of the drug delivery management device 106. Based on the information determined from the indicator 206, a particular version of software can be selected and configured for managing drug delivery operations of the drug delivery device 102 through the drug delivery management device 106. In various embodiments, the drug delivery management device 106 can identify a particular version of software for controlling operation of the drug delivery device 102 that is appropriate based on the information determined from the indicator 206. The identified software can subsequently be downloaded or provided to the drug delivery management device 106 remotely - for example, from the cloud platform 114.

As an example, the drug container 202 can include U-100 insulin as the drug 204. The indicator 206 can include an indication that the stored drug 204 is U-100 insulin. When the type of the drug 204 is determined based on, for example, scanning or capturing an image of the indicator 206, the appropriate software for controlling operation of the drug delivery device 102 by the drug delivery management device 106 for delivering U-100 insulin can be determined. The identified appropriate software can then be configured on the drug delivery management device 106 - for example, either by retrieving the software and storing it on the drug delivery management device 106 for execution or by identification of pre-loaded/pre-stored software already resident on the drug delivery management device 106.

If the drug container 202 includes U-200 insulin as the drug 204, then different appropriate software can be configured for controlling operation on the drug delivery device 102 by the drug delivery management device 106 in a similar manner. By enabling the configuration of the drug delivery management device 106 to vary based on the type of the drug 204 within the drug container 202 (and/or based on other information provided by the indicator 206), the same drug delivery management device 106 (and/or drug delivery device 102) can be used for drug delivery for a variety of different drugs.

Specifically, the drug delivery management device 106 is not limited to a single configuration tied to a single type of drug and a single type of drug delivery device 102. Instead, the drug delivery management device 106 can be configured with different software appropriate for each different type of drug that can be loaded and used by the drug delivery management device 106. The different software can control the drug delivery by the drug delivery device 102 differently for each drug type - for example, by varying dosage sizes and/or times as appropriate. As a result, the need for a different type of drug delivery management device 106 for each different type of drug is no longer necessary, thereby increasing the flexibility of the drug delivery system 100 and reducing its cost as fewer drug delivery management device 106 SKUs are needed.

In various embodiments, the drug delivery management device 106 can include components and/or mechanisms (e.g., a bar code scanner and/or a camera) for capturing information provided by the indicator 206. In various embodiments, the captured information from the indicator 206 can be processed by the drug delivery management device 106 to determine the appropriate configuration of the drug delivery management device 106. In various embodiments, the drug delivery management device 106 can provide the captured information (e.g., the scanned bar code information and or an image of the indicator 206) to the cloud platforms 114 enabling remote processing of the captured information for determination of appropriate configuration.

The drug delivery management device 106 is not limited to use of a bar code scanner and/or a camera for capturing information relating to the drug container 202. In various embodiments, information can be exchanged wirelessly (e.g., automatically) between the drug container 202 and the drug delivery management device 106. For example, the drug container 202 can contain a radio-frequency identification (RFID) tag that can be scanned by an RFID reader of the drug delivery management device 106. Other examples for exchanging information can include Wi-Fi links, near field communication links, Bluetooth links, and/or any other short range wireless communication protocols that may include automatic discovery exchanges of information.

In various embodiments, configuration of the drug delivery management device 106 can be customized to a particular user. For example, connectivity to the cloud platforms 114 can enable user information (e.g., patient information, drug use history, medical condition history, etc.) to be used to enable further additional configuration of the drug delivery management device 106 and/or any component of the drug delivery system 100.

In various embodiments, the drug delivery management device 106 can include one or more processors, a memory component, and a transceiver. The transceiver can enable data or other information to be communicated to or from the drug delivery management device 106. As an example, the transceiver can send information indicated by the indicator 206 to the cloud platform 114. The transceiver can also receive operational software for the drug delivery management device 106 from the cloud platform 114. The memory can store computer code that can be executed by the processor. The processor can execute stored computer code to direct operation of the drug delivery management device 106 and, in turn, operation of the drug delivery device 102. In various embodiments, the memory component can store different versions of operational software for the drug delivery management device 106. A particular version of the operational software can be selected for implementation by the drug delivery management device 106 based on information from the indicator 206.

In various embodiments, the techniques described herein provide redundant safety mechanism for the user. In various embodiments, manual configuration of the drug delivery management device 106 can be confirmed based on information from the indicator 206 as processed by the drug delivery system 100. For example, a juvenile user may attempt to load the drug delivery device 102 with U-500 insulin. If the juvenile user is not authorized to use U-500 insulin, then processing the information from the indicator 206 can enable the drug delivery management device 106 to be configured to prevent drug delivery operations of the drug delivery device 102.

In various other embodiments, an image of the indicator 206 and/or the drug 204 provides an opportunity to confirm the drug 204 is not expired or recalled. Further, warnings based on the quality of the drug 204 - e.g., based on a check of the color or discoloration of the drug 204 - can be provided. In various embodiments, alerts, warnings, or other alarms can be provided to the user by the drug delivery system 100. As an example, alarms can be provided to the user by the drug delivery management device 106. Alarms can include any visual, audible, or haptic (e.g., vibrational) alarm.

In various embodiments, information from the indicator 206 can be used to verify that a user is approved to use the drug 204. For example, the user may be associated with one or more drugs that the user is approved to use - e.g., a configuration list of approved drugs. If the information from the indicator 206 indicates that the drug 204 is not an approved drug, then the drug delivery management device 106 and/or the drug delivery system 100 can provide a warning or alert to the user. Further, the drug delivery management device 106 and/or the drug delivery system 100 can prevent operation or use if the user attempts to use the unapproved drug.

In various embodiments, the drug delivery management device 106 may include location determination features (e.g., GPS location identification features). Accordingly, the drug delivery management device 106 can lock or unlock certain operational settings or configurations of the drug delivery management device 106 based on the location of the drug delivery management device 106. For example, the drug delivery management device 106 may determine that a certain drug cannot be used in certain regions or countries, and therefore can prevent use of drugs not approved for certain regions or zones. As another example, location information can enable the drug delivery management device 106 to provide the user with information such as, for example, location proximity to therapy centers, distribution centers, pharmacies, repair locations, etc.

As previously mentioned, the drug delivery management device 106 can be a smartphone or other personal electronic device. For example, as a smartphone, the drug delivery management device 106 can download and configure the appropriate application (app) based on information provided by processing the indicator 206 as discussed herein.

In various embodiments, the drug delivery management device 106 can store two or more different software programs for controlling operation of the drug delivery management device 106. The different software programs can each correspond to a different use or control of the drug delivery device 102 to deliver a drug to the user - for example, each software program can correspond to a particular drug type, drug dosage, and/or drug concentration. The software programs can each be stored on the drug delivery management device 106 and a particular software program can be identified for use based on the determined information relating to the drug. As an alternative to storing the different software programs on the drug delivery management device 106, the software programs can be stored remotely (e.g., by the cloud platform 114). Once the proper software program is identified, the identified software program can be transmitted (e.g., wirelessly) to the drug delivery management device 106 from the cloud platform 114.

Under either scenario, the drug delivery management device 106 can capture and process information relating to the drug to be delivered to the user (e.g., by collecting such information from the drug delivery device 102 and/or a container storing the drug). After processing the information, a particular software program for controlling operation of the drug delivery management device 106 (and/or any other component of the drug delivery system 100) can be determined or identified. The identified software program can then be configured to control operation of the drug delivery management device 106. As a result, a software program and a particular configuration for operation of the drug delivery management device 106 (and/or any other component of the drug delivery system 100) can be provided for a specific application or intended use of the drug delivery system 100.

In various embodiments, the information relating to the drug to be delivered to the user can be provided by an indicator coupled to the drug container (e.g., the indicator 206 coupled to the container 202) or can be provided by an indicator (e.g., a bar code, QR code, RFID tag, and/or a visual indicator) coupled to the drug delivery device 104. In various embodiments, a software program and/or a configuration of operation of the drug delivery device 102 can also be provided based on the determined information relating to the drug to be provided to the user. For example, once a drug type, dose, or concentration of the drug is determined, a software program and/or configuration of operation of the drug delivery device 102 can also be provided through interaction with the cloud platform 114 and/or the drug delivery management device 106.

In various embodiments, the software for controlling operation of the drug delivery management device 106 (and/or the software for controlling operation of any component of the drug delivery system 100) can be any computer readable and executable code including an operating system, user interface, firmware, and/or an application specific program.

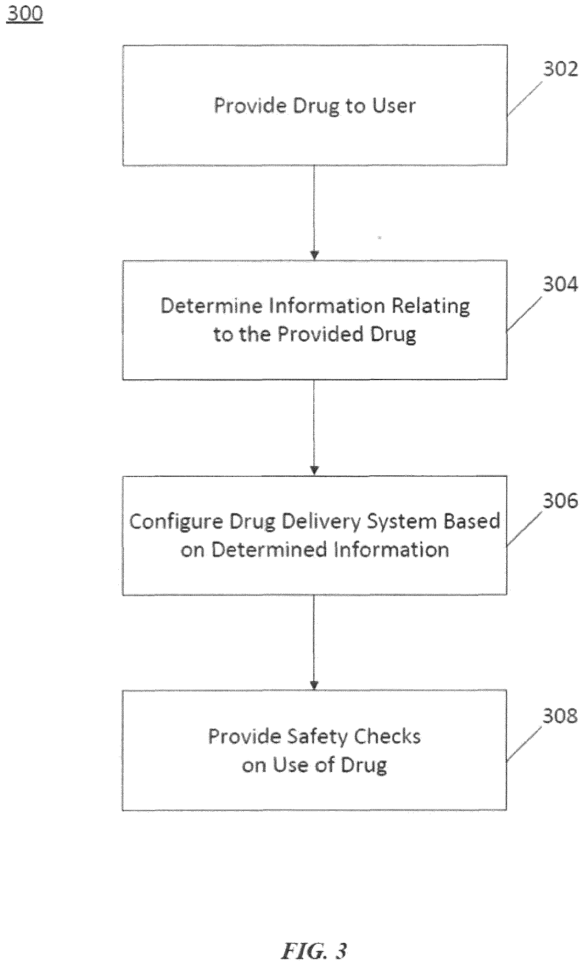

FIG. 3 illustrates an exemplary method of operation 300 for a drug delivery system. The method of operation 300 can be implemented by the drug delivery system 100.

At 302, a drug can be provided to a user or patient. The patient can also be provided with (or can already be in possession of) a drug delivery device (e.g., the drug delivery device 102) and a drug delivery management device (e.g., the drug delivery management device 106). The drug can be provided to the user within a drug vial (e.g., the drug vial 202) or within a drug cartridge (e.g., an ISO drug cartridge).

At 304, information related to the provided drug can be determined. Information related to the drug can be contained, for example, on an indicator (e.g., the indicator 206) provided on a container storing the drug. The indicator can be a bar code or a QR code but is not so limited. The indicator can include any visual, graphical, textual, or coded information. The information relating to the drug can be captured by the drug delivery management device used by the patient. The drug delivery management device can contain, for example, a bar code scanner, camera, or other device for capturing information provided by the indicator. The captured information can be processed by the drug delivery system. In various embodiments, one or more components of the drug delivery system can review any captured image of the indicator and/or can decode any coded bar code information of the indicator to determine information relating to the provided drug. Information relating to the drug can include the type of drug, dosing recommendations, drug concentration, manufacturer, prescription information, drug provider, etc.

At 306, the drug delivery system can be configured based on any information determined at 304. One or more components of the drug delivery system - e.g., the drug delivery device, a management device, and/or a sensor - can be configured or managed based on the determined information. As an example, software for the management device can be determined and/or configured. In various embodiments, the software can be stored on the management device and can be identified, unlocked, and/or configured for use. In other embodiments, the software can be downloaded to the management device from a remote device (e.g., a remote cloud platform 114). In other embodiments, particular features of stored software can be locked or unlocked. The configured software can then be used to direct operation of the drug delivery device to deliver the drug to the user.

At 308, the drug delivery system can provide the user with any warnings or alerts regarding the drug or use thereof. For example, the drug delivery system can confirm whether or not the drug is expired or the subject of any recalls. The drug delivery system can confirm that the patient is allowed to use the particular drug based on, for example, any age, use, or location restrictions related to use of the drug. The drug delivery system can issue an alarm or alert to the user through the management device - for example, using a visual, audible, or haptic alarm.

Many conventional drug delivery devices are intended to be of low cost and complexity. For example, some conventional drug delivery devices may not include any user input components or features (e.g., a button to initiate needle insertion and drug delivery), an output display, or any communication capabilities.

Many such conventional devices are intended to be filled with a drug by a user or a health care specialist which, once filled, triggers a timer. When the timer expires, the drug delivery device is automatically activated - for example, a needle may be intended to be inserted into the user and drug delivery is to begin. Many users or health care workers may find this automatic activation to be very restrictive in that activation cannot be delayed or paused. Tying activation of these conventional drug delivery devices to a non-adjustable timer can result in rushed or erroneous placement of the drug delivery device, or may result in the device being activated before proper placement is achieved.

In various embodiments, the drug delivery device 102 can be a drug delivery device that is intended to be of relatively low complexity and cost. As an example, the drug delivery device 102 can be designed to not include an output display or any input component or device (e.g., a button or other user input feature). Techniques described herein can expand the capabilities of such an embodiment of the drug delivery device 102 without greatly increasing the cost and complexity of the drug delivery device 102.

In various embodiments, the drug delivery device 102 - as a relatively low cost and low complexity device - can maintain the same size, shape, and/or form factor while providing additional user control and information sharing capabilities. By maintaining the same size, shape, and/or form factor (e.g., by not having to introduce a user input feature or output display feature), the cost of improving the user control and information sharing capabilities can be minimized. In various embodiments, the drug delivery device 102 can be fitted with one or more electronic components to facilitate control by a management device - for example, the drug delivery management device 106 - and to enable operational data to be offloaded.

In various embodiments, the one or more electronic components of the drug delivery device 102 can enable the drug delivery management device 106 to start, stop, and/or pause activation of the drug delivery device 102. For example, a timer that is triggered when the drug delivery device 102 is filled with a liquid drug can be controlled to stop or be paused by wireless control from the drug delivery management device 106.

In various embodiments, operational data of the drug delivery device 102 can be offloaded for storage and/or analysis. This operational data can include such information as the amount of drug delivered over a period of time, the amount of drug remaining, the type of the drug delivery device 102, the drug contained in the drug delivery device, etc. The operational data can be wirelessly transmitted to the drug delivery management device 106 or the cloud platform 114.

In various embodiments, the drug delivery device 102 can include one or more electronic components to facilitate wireless communication through any known standard or protocol such as, for example, NFC, Wi-Fi, Bluetooth, or any cellular standard. In various embodiments, the drug delivery management device 106 can include a bar code scanner or a camera for reading or capturing identification information of the drug delivery device 102. For example, the drug delivery device 102 may include a bar code or QR code that identifies the type of device, type of drug, etc. The drug delivery management device 106 can read and process the bar code or QR code and can then be configured to interact with the drug delivery device 102. Once configured to operate with the drug delivery device 102, the drug delivery management device 106 can allow a user to better control initiation of the drug delivery device 102.

In various embodiments, with the inclusion of electronic components to facilitate communications, the drug delivery device 102 can be connected to the cloud platform 114. As a result, the drug delivery device 102 can be remotely authenticated, use can be tracked and timestamped, location can be tracked (e.g., though GPS tracking), and software on the device can be loaded or updated. Further, data regarding the operation or operational state of the drug delivery device 102 can be relayed and provided to the cloud platform 114 for storage and/or analysis. The offloaded data can be synched with patient data for more robust analysis on the effectiveness of the drug delivery device 102.

Certain embodiments of the present invention were described above. It is, however, expressly noted that the present invention is not limited to those embodiments, but rather the intention is that additions and modifications to what was expressly described herein are also included within the scope of the invention. Moreover, it is to be understood that the features of the various embodiments described herein were not mutually exclusive and can exist in various combinations and permutations, even if such combinations or permutations were not made express herein, without departing from the spirit and scope of the invention. In fact, variations, modifications, and other implementations of what was described herein will occur to those of ordinary skill in the art without departing from the spirit and the scope of the invention. As such, the invention is not to be defined only by the preceding illustrative description.

## Claims

1. A drug delivery system, comprising:
a drug delivery device configured to store and deliver a drug to a user; and
a drug delivery management device configured to manage operation of the drug delivery device, the drug delivery management device configured to determine information relating to the drug based on an indicator coupled to a container configured to store the drug and to determine a software program for controlling operation of the drug delivery management device based on the determined information relating to the drug.

2. The drug delivery system of claim 1, wherein the drug delivery management device includes one or more of a bar code scanner, a quick response (QR) code scanner, and a camera.

3. The drug delivery system of claim 2, wherein the indicator comprises a QR code.

4. The drug delivery system of claim 2, wherein the indictor comprises a bar code.

5. The drug delivery system of claim 1, wherein the drug delivery management device includes a radio-frequency identification (RFID) scanner.

6. The drug delivery system of claim 5, wherein the indicator comprises an RFID tag.

7. The drug delivery system of claim 1, wherein the information comprises at least one of a type of the drug, a dosage of the drug, and a concentration of the drug.

8. The drug delivery system of claim 7, wherein the drug delivery management device is configured to provide an alarm to the user when the information indicates that the user is not approved for the drug.

9. The drug delivery system of claim 1, wherein the container comprises at least one of a drug vial and an International Organization for Standardization (ISO) cartridge.

10. The drug delivery system of claim 1, wherein the determined software program is stored on the drug delivery management device and is selected from among a plurality of software programs stored on the drug delivery management device.

11. The drug delivery system of claim 1, wherein the determined software program is stored remotely and is transmitted wirelessly to the drug delivery management device.

12. The drug delivery system of claim 1, wherein the drug delivery management device is configured to manage operation of the drug delivery device based on a determined location of the drug delivery device.

13. The drug delivery system of claim 1, wherein the drug delivery management device is configured to provide an alarm to the user based on an identified safety issue relating to the drug.

14. The drug delivery system of claim 1, wherein operation of the determined software program is customized based on information relating to the user.

15. A method, comprising:
providing to a user a container storing a drug;
determining information relating to the drug;
configuring operation of a drug delivery management device based on the determined information relating to the drug; and
managing operation of a drug delivery device based on the configuration of the drug delivery management device to deliver the drug to the user.

16. The method of claim 15, wherein determining comprises scanning one of a bar code or a quick response (QR) code coupled to the container.

17. The method of claim 15, wherein determining comprises scanning a radio-frequency identification (RFID) tag coupled to the container.

18. The method of claim 15, wherein determining comprising capturing an image of an indicator coupled to the container.

19. The method of claim 15, wherein configuring comprises identifying a software program for controlling operation of the drug delivery management device.

20. The method of claim 19, further comprising selecting the identified software program from a plurality of software programs stored on the drug delivery management device.

21. The method of claim 19, further comprising receiving the identified software program from a remote computing device.

22. The method of claim 15, further comprising providing an alarm to the user based on a determined safety issue relating to the drug.

23. The method of claim 22, further comprising providing the alarm to the user when the information indicates the user is not authorized to use the drug.

24. The method of claim 22, wherein the alarm is one of a visual, an audible, or a vibrational alarm.
